(19) Europäisches Patentamt
European Patent Office
Office européen des brevets

(11) Veröffentlichungsnummer: **0 350 707 A1**

(12) # EUROPÄISCHE PATENTANMELDUNG

(21) Anmeldenummer: 89111653.5

(51) Int. Cl.4: C07J 1/00 , A61K 31/565

(22) Anmeldetag: 27.06.89

(30) Priorität: 13.07.88 DE 3824247

(43) Veröffentlichungstag der Anmeldung: 17.01.90 Patentblatt 90/03

(84) Benannte Vertragsstaaten: AT BE CH DE ES FR GB GR IT LI LU NL SE

(71) Anmelder: SCHERING AKTIENGESELLSCHAFT
Berlin und Bergkamen
Müllerstrasse 170/178 Postfach 65 03 11
D-1000 Berlin 65(DE)

(72) Erfinder: Bittler, Dieter
Bölkauer Pfad 11
D-1000 Berlin 27(DE)
Erfinder: Laurent, Henry, Dr.
Glambecker Weg 21
D-1000 Berlin 28(DE)
Erfinder: Rach, Petra
Antwerpener Strasse 1
D-1000 Berlin 65(DE)
Erfinder: Töpert, Michael, Dr.
Sigismundkorso 58
D-1000 Berlin 28(DE)

(54) **Neue Androstan-Derivate.**

(57) Androstan-Derivate der allgemeinen Formel I

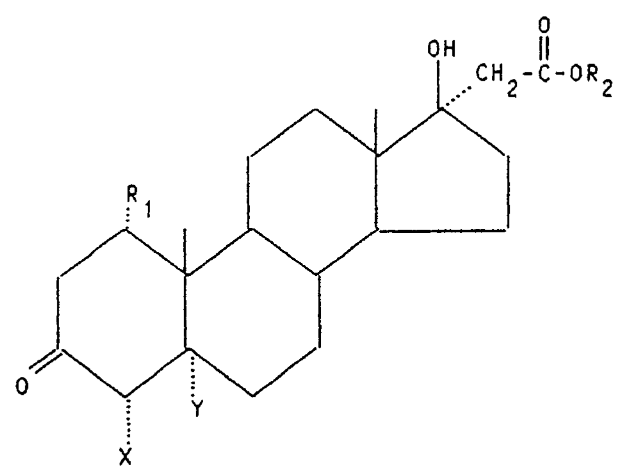

(I),

worin
$R_1$ ein Wasserstoffatom oder eine Methylgruppe darstellt,
X und Y jeweils Wasserstoffatome oder gemeinsam eine Kohlenstoff-Kohlenstoffbindung bedeuten und
$R_2$ ein Wassestoffatom oder eine Alkylgruppe mit 1 bis 6 Kohlenstoffatomen symbolisiert, sind pharmakologisch wirksame Substanzen, die bei topischer Applikation eine ausgeprägte antiandrogene Wirksamkeit besitzen.

EP 0 350 707 A1

## Neue Androstan-Derivate

Die Erfindung betrifft neue Androstan-Derivate der allgemeinen Formel I

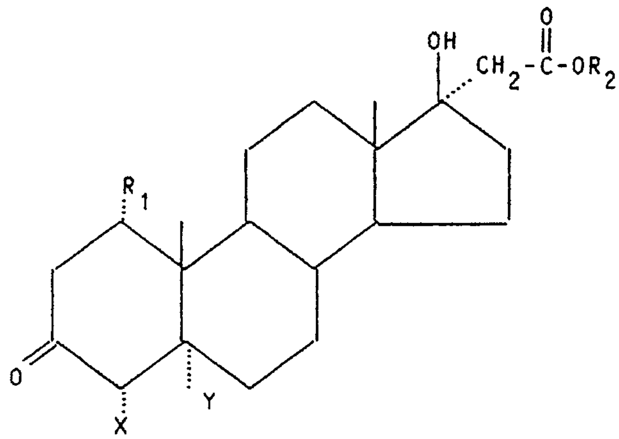

(I),

worin

$R_1$ ein Wasserstoffatom oder eine Methylgruppe darstellt,

X und Y jeweils Wasserstoffatome oder gemeinsam eine Kohlenstoff-Kohlenstoffbindung bedeuten und

$R_2$ ein Wassestoffatom oder eine Alkylgruppe mit 1 bis 6 Kohlenstoffatomen symbolisiert und pharmazeutische Präparate, die ein oder zwei dieser Androstan-Derivate als Wirkstoff enthalten.

Unter einem Alkylrest $R_2$ soll ein geradkettiger oder verzweigter gesättigter alicyclischer Kohlenwasserstoffrest mit 1 bis 6 Kohlenstoffatomen verstanden werden. Als geeignete Alkylreste seien beispielsweise genannt:

Der Methylrest, der Ethylrest, der Propylrest, der Isopropylrest, der Butylrest, der 2-Butylrest, der 2-Methylpropylrest, der tert.-Butylrest, der Pentylrest oder der Hexylrest.

Die Erfindung betrifft ferner ein Verfahren zur Herstellung von Androstan-Derivaten der allgemeinen Formel Ia

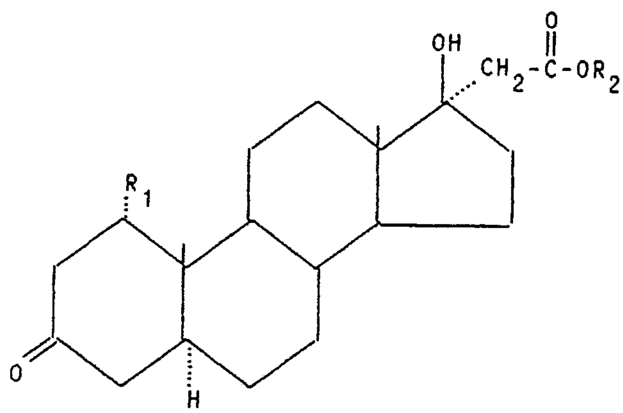

(Ia),

worin $R_1$ und $R_2$ die oben genannte Bedeutung besitzen, welches dadurch gekennzeichnet ist, daß man man ein Steroid-Ketal der allgemeinen Formel II

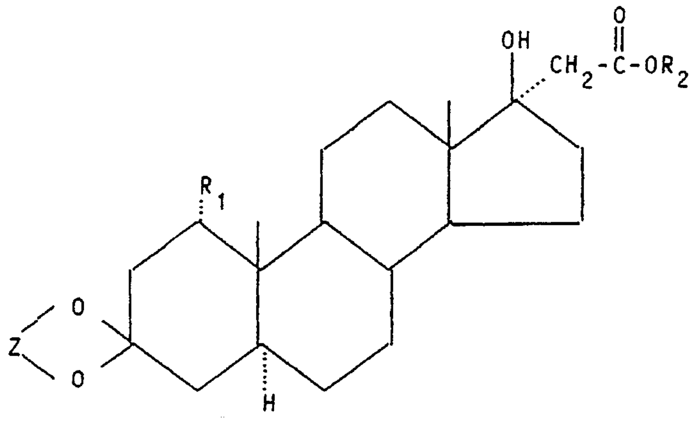

(II),

worin $R_1$ und $R_2$ die oben genannte Bedeutung besitzen und Z eine Alkylengruppe mit 1 bis 6 Kohlenstoffatomen darstellt, hydrolytisch spaltet und gewünschtenfalls die 17α-Alkoxycarbonylverbindungen der allgemeinen Formel Ia verseift und/oder die Carbonsäuren der allgemeinen Formel Ia verestert.

Letztlich betrifft die Erfindung ein Verfahren zur Herstellung von Androstan-Derivaten der allgemeinen Formel Ib

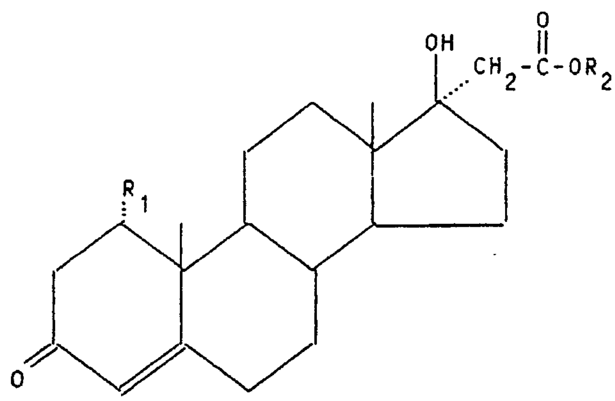

(Ib),

worin $R_1$ und $R_2$ die oben genannte Bedeutung besitzen, welches dadurch gekennzeichnet ist, daß man einen Enolether der allgemeinen Formel III

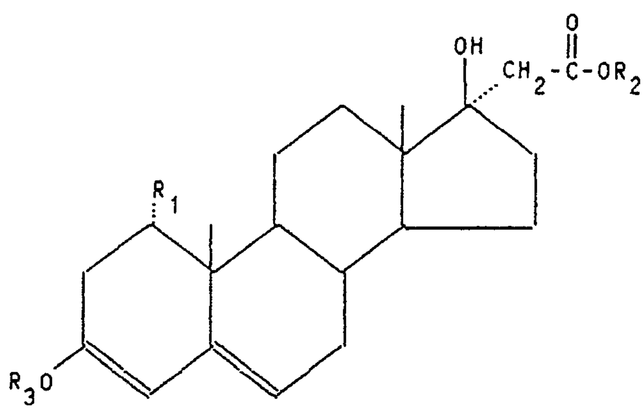

(III),

worin $R_1$ und $R_2$ die oben genannte Bedeutung besitzen und $R_3$ eine Alkylgruppe mit 1 bis 6 Kohlenstoffatomen darstellt, oder ein Ketal der allgemeinen Formel IV

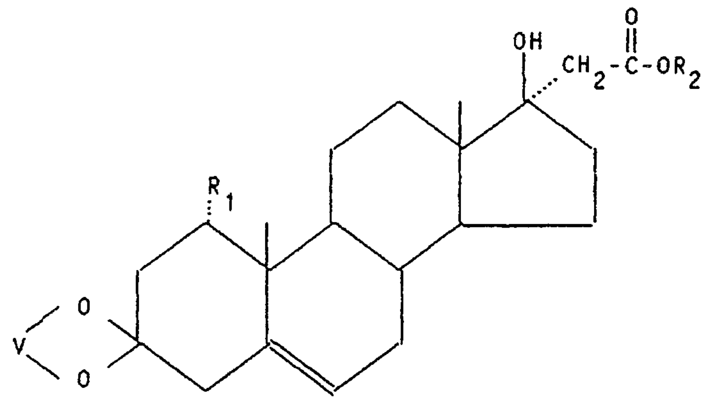

(IV),

worin $R_1$ und $R_2$ die oben genannte Bedeutung besitzen und V eine Alkylengruppe mit 1 bis 6 Kohlenstoffatomen darstellt hydrolytisch spaltet und gewünschtenfalls die 17α-Alkoxycarbonylverbindungen der allgemeinen Formel Ib verseift und/oder die Carbonsäuren der allgemeinen Formel Ib verestert.

Die Durchführung des erfindungsgemäßen Verfahrens erfolgt unter Bedingungen, die dem Fachmann wohlbekannt sind. So kann man beispielsweise die Spaltung der Ketale und Enolether unter den Bedingungen durchführen, die in dem Buch von Carl Djerassi "Steroid Reactions"; Holden-Day Inc.; San Francisco 1963 beschrieben werden. Die Verseifung der Alkoxycarbonylverbindungen und die Veresterung der Carbonsäuren läßt sich beispielsweise unter den im US-Patent 3,824,260 beschriebenen Bedingungen durchführen.

Die Ausgangsverbindungen für das erfindungsgemäße Verfahren können aus den entsprechenden 17-Ketoverbindungen hergestellt werden, indem man diese beispielsweise unter den Bedingungen, wie sie in den nachfolgenden Ausführungsbeispielen erwähnt sind mit Bromessigsäurealkylestern alkyliert.

Die Verbindungen der allgemeinen Formel Ia und diejenigen der allgemeinen Formel I b (von denen bislang allerdings nur solche Verbindungen näher untersucht wurden, die als Substituenten $R_1$ ein Wasserstoffatom tragen) zeigen bei topischer Applikation eine ausgeprägte antiandrogene Wirksamkeit sind aber bei systemischer Applikation nur gering wirksam.

Zur topischen Anwendung können die erfindungsgemäßen Verbindungen mit den üblichen Trägersubstanzen zu Lösungen, Gels, Salben, Pudern oder anderen Präparaten verarbeitet werden. Geeignete Tägersubstanzen sind zum Beispiel Wasser, Ethanol, Propanol, Glycerin, Methylcellulose, Hydroxypropylcellulose, Carboxymethylen usw.. Das Antiandrogen liegt vorzugsweise in einer Konzentration von 0,05 bis 5,0 Gewichtsprozent, bezogen auf das Gesamtgewicht des Präparates, vor. Die Präparate können zur topischen Behandlung von Erkrankungen wie Akne, Seborrhoe, Alopezie und Hirsutismus verwendet werden.

Die nachstehenden Ausführungsbeispiele dienen zur näheren Erläuterung des erfindungsgemäßen Verfahrens.

BEISPIEL 1

a) Eine Lösung von 450 mg Kupfer(II)-acetat in 111 ml Ethylenglykoldimethylether wird mit 22.5 g Zinkstaub und 2.15 ml Essigsäure versetzt und bis zur Entfärbung der Lösung bei Raumtemperatur gerührt. Zur Reaktionsmischung werden 0.75 ml Triethylamin und 15 g 3,3-Ethylendioxy-1α-methyl-5α-androstan-17-on (hergestellt gemäß EP-A 71153) zugegeben und innerhalb 90 min 50.25 ml Ethylbromacetat zugetropft. Anschließend wird 2 h bei Raumtemperatur gerührt und das überschüssige Reagenz mit gesättigter Ammoniumchloridlösung zersetzt. Nach Zugabe von Diethylether wird die wäßrige Phase abgetrennt und die organische Phase mit Ammoniumchloridlösung und Wasser gewaschen. Nach dem Trockenen und Eindampfen wird der Rückstand an Kieselgel chromatographiert, und man erhält 12.5 g 3,3-Ethylendioxy-17α-ethoxycarbonylmethyl-1α-methyl-5α-androstan-17β-ol.

b) Eine Lösung von 4.2 g 3,3-Ethylendioxy-17α-ethoxycarbonylmethyl-1α-methyl-5α-androstan-17β-ol in 42 ml Methanol und 21 ml Tetrahydrofuron wird mit 4.2 ml 8%iger Schwefelsäure versetzt und 90 min bei Raumtemperatur stehengelassen. Das Reaktionsgemisch wird mit Diethylether verdünnt, mit Wasser neutral gewaschen, getrocknet und eingedampft. Der Rückstand wird an Kieselgel chromatographiert. Man isoliert 3.2 g 17α-Ethoxycarbonylmethyl-17β-hydroxy-1α-methyl-5α-androstan-3-on als zähes Öl.

BEISPIEL 2

Eine Lösung von 3.9 g 17α-Ethoxycarbonylmethyl-17β-hydroxy-1α-methyl-5α-androstan-3-on in 88 ml Methanol und 58 ml Wasser wird mit 9.7 ml 2N Natronlauge ver setzt und 2.5 h bei Raumtemperatur gerührt. Die Reaktionslösung wird mit Wasser verdünnt, zweimal mit Diethylether extrahiert und mit 12 ml 2N Schwefelsäure angesäuert. Die freigesetzte Carbonsäure wird in Dichlormethan aufgenommen, die organische Phase mit Wasser gewaschen, getrocknet und eingedampft. Man erhält 3.3 g 17α-Carboxymethyl-17β-hydroxy-1α-methyl-5α-androstan-3-on als öliges Produkt.

BEISPIEL 3

Eine eisgekühlte Lösung von 700 mg 17α-Carboxymethyl-17β-hydroxy-1α-methyl-5α-androstan-3-on in 15 ml Tetrahydrofuran wird mit 10 ml einer etherischen Diazomethanlösung (hergestellt aus 700 mg Nitrosomethylharnstoff) versetzt und 30 min bei Eiskühlung stehengelassen. Die Lösung wird im Vakuum zur Trockne eingedampft und der Rückstand an Kieselgel chromatographiert. Nach dem Umkristallisieren aus Diisopropylether werden 340 mg 17β-Hydroxy-17α-methoxycarbonylmethyl-1α-methyl-5α-androstan-3-on vom Schmelzpunkt 119.5 °C erhalten.

BEISPIEL 4

Eine Lösung von 750 mg 17α-Carboxymethyl-17β-hydroxy-1α-methyl-5α-androstan-3-on in 7.5 ml Dimethylformamid wird nach Zugabe von 0.52 ml 1-Brompropan und 520 mg Silberoxid 1.5 h bei 60 °C gerührt. Anschließend wird mit Diethylether verdünnt, mit Wasser gewaschen, getrocknet und eingedampft. Der Rückstand wird an Kieselgel chromatographiert, und es werden 710 mg 17β-Hydroxy-1α-methyl-17α-propoxycarbonylmethyl-5α-androstan-3-on als Öl erhalten.

BEISPIEL 5

Eine Lösung von 630 mg 17a-Carboxymethyl-17β-hydroxy-1α-methyl-5α-androstan-3-on in 5.8 ml Dimethylformamid wird mit 0.488 ml 1-Brombutan und 438 mg Silberoxid versetzt und 1.5 h bei 60 °C gerührt. Es wird, wie in Beispiel 4 beschrieben, aufgearbeitet und chromatographiert. Man erhält 430 mg öliges 17α-Butoxycarbonylmethyl-17β-hydroxy-1α-methyl-5α-androstan-3-on.

BEISPIEL 6

10 g 3-Methoxy-3,5-androstadien-17-on (hergestellt gemäß J. Org. Chem. 26 (1961) 3925) werden, wie im Beispiel 1 beschrieben, in Ethylenglykoldimethylether mit Zink-Kupfer und Ethylbromacetat umgesetzt und aufgearbeitet. Nach Chromatographie an Kieselgel werden 9 g ethylbromacetathaltiges 17α-Ethoxycarbonylmethyl-17β-hydroxy-3-methoxy-3,5-androstadien erhalten, das in 90 ml Methanol und 45 ml Tetrahydrofuran gelöst wird. Die Lösung wird nach Zugabe von 9 ml 8%iger Schwefelsäure bei Raumtemperatur stehengelassen, mit Diethylether verdünnt und mit Wasser neutral gewaschen und getrocknet. Das Lösungsmittel wird i. Vak. verdampft und der Rückstand an Kieselgel chromatographiert. Nach dem Umkristallisieren aus Diisopropylether erhält man 1.6 g 17α-Ethoxycarbonylmethyl-17β-hydroxy-4-androsten-3-on vom Schmelzpunkt 88 °C.

BEISPIEL 7

Eine Lösung von 1.1 g 17α-Ethoxycarbonylmethyl-17β-hydroxy-4-androsten-3-on in 30 ml Methanol und 16.3 ml Wasser wird mit 3.73 ml 2N Natronlauge versetzt und 2 h bei Raumtemperatur stehengelassen. Die Reaktionslösung wird mit Wasser verdünnt, zweimal mit Diethylether extrahiert und mit 4.5 ml 2N Schwefelsäure angesäuert. Die entstandene Carbonsäure wird mit Dichlormethan aufgenommen, die organische Phase wird mit Wasser gewaschen, getrocknet und eingedampft. Es werden 920 mg 17α-Carboxymethyl-17β-hydroxy-4-androsten-3-on als Rohprodukt erhalten.

BEISPIEL 8

400 mg 17α-Carboxymethyl-17β-hydroxy-4-androsten-3-on werden mit etherischer Diazomethanlösung in Tetrahydrofuran, wie im Beispiel 3 beschrieben, umgesetzt und aufgearbeitet. Man erhält nach Chromatographie und Kristallisation aus Diisopropylether 225 mg 17β-Hydroxy-17α-methoxycarbonylmethyl-4-androsten-3-on vom Schmelzpunkt 114 °C.

BEISPIEL 9

300 mg 17α-Carboxymethyl-17β-hydroxy-4-androsten-3-on werden, wie im Beispiel 4 beschrieben, mit Silberoxid und 1-Brompropan umgesetzt und aufgearbeitet. Nach Chromatographie an Kieselgel werden 180 mg 17β-Hydroxy-17α-propoxycarbonylmethyl-4-androsten-3-on vom Schmelzpunkt 130 °C erhalten.

## Ansprüche

1. Androstan-Derivate der allgemeinen Formel I

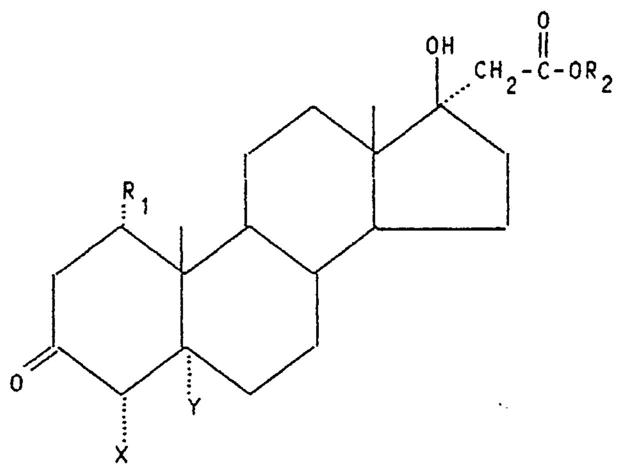

(I),

worin
$R_1$ ein Wasserstoffatom oder eine Methylgruppe darstellt,
X und Y jeweils Wasserstoffatome oder gemeinsam eine Kohlenstoff-Kohlenstoffbindung bedeuten und
$R_2$ ein Wassestoffatom oder eine Alkylgruppe mit 1 bis 6 Kohlenstoffatomen symbolisiert.

2. 17α-Ethoxycarbonylmethyl-17β-hydroxy-1α-methyl-5α-androstan-3-on.
3. 17α-Carboxymethyl-17β-hydroxy-1α-methyl-5α-androstan-3-on.
4. 17β-Hydroxy-17α-methoxycarbonylmethyl-1α-methyl-5α-androstan-3-on.
5. 17β-Hydroxy-1α-methyl-17α-propoxycarbonylmethyl-5α-androstan-3-on.
6. 17α-Butoxycarbonylmethyl-17β-hydroxy-1α-methyl-5α-androstan-3-on.
7. 17α-Ethoxycarbonylmethyl-17β-hydroxy-4-androsten-3-on.
8. 17α-Carboxymethyl-17β-hydroxy-4-androsten-3-on.
9. 17β-Hydroxy-17α-methoxycarbonylmethyl-4-androsten-3-on.
10. 17β-Hydroxy-17α-propoxycarbonylmethyl-4-androsten-3-on.
11. Pharmazeutische Präparate enthaltend ein oder zwei Androstan-Derivate gemäß Patentanspruch 1 bis 10 als Wirkstoff.
12. Verfahren zur Herstellung von Androstan-Derivaten der allgemeinen Formel Ia

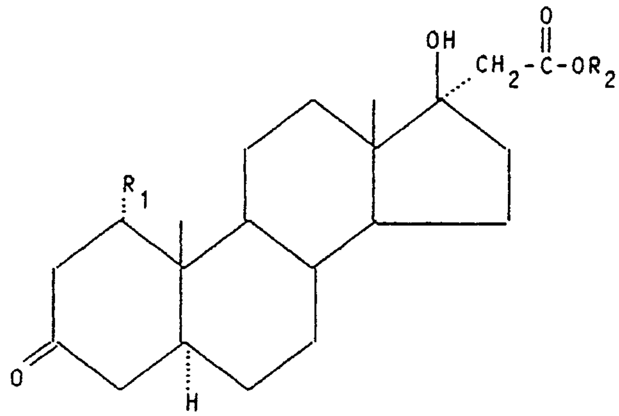

(Ia),

worin $R_1$ und $R_2$ die im Anspruch 1 genannte Bedeutung besitzen, dadurch gekennzeichnet, daß man ein Steroid-Ketal der allgemeinen Formel II

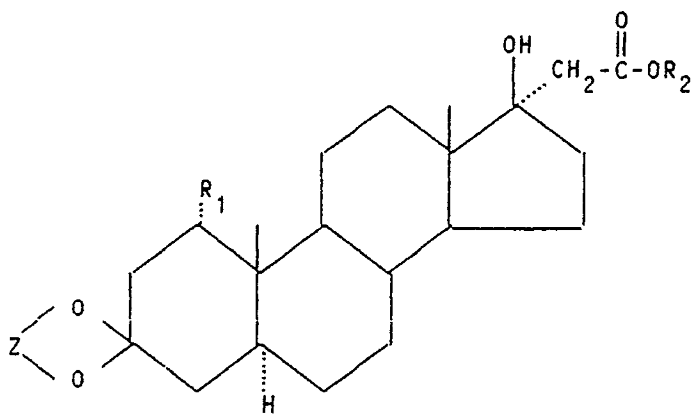

(II),

worin $R_1$ und $R_2$ die oben genannte Bedeutung besitzen und Z eine Alkylengruppe mit 1 bis 6 Kohlenstoffatomen darstellt, hydrolytisch spaltet und gewünschtenfalls die 17α-Alkoxycarbonylverbindungen der allgemeinen Formel Ia verseift und/oder die Carbonsäuren der allgemeinen Formel Ia verestert.

13. Verfahren zur Herstellung von Androstan-Derivaten der allgemeinen Formel Ib

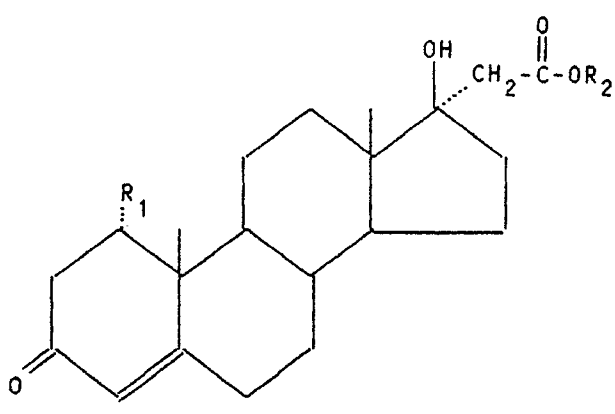

(Ib),

worin $R_1$ und $R_2$ die im Anspruch 1 genannte Bedeutung besitzen, dadurch gekennzeichnet, daß man einen Enolether der allgemeinen Formel III

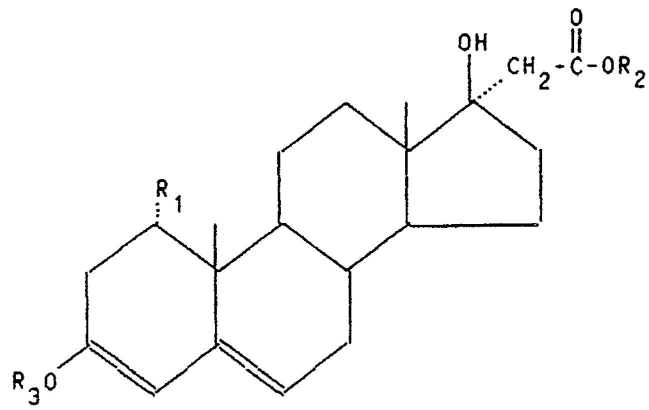

(III),

worin $R_1$ und $R_2$ die oben genannte Bedeutung besitzen und $R_3$ eine Alkylgruppe mit 1 bis 6 Kohlenstoffatomen darstellt, oder ein Ketal der allgemeinen Formel IV

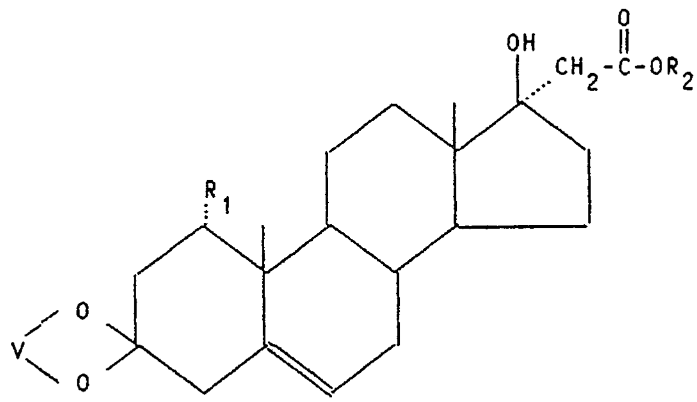

(IV),

worin $R_1$ und $R_2$ die oben genannte Bedeutung besitzen und V eine Alkylengruppe mit 1 bis 6 Kohlenstoffatomen darstellt hydrolytisch spaltet und gewünschtenfalls die 17α-Alkoxycarbonylverbindungen der allgemeinen Formel Ib verseift und/oder die Carbonsäuren der allgemeinen Formel Ib verestert.

Europäisches Patentamt

# EUROPÄISCHER RECHERCHENBERICHT

Nummer der Anmeldung

EP 89 11 1653

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 099 854 (SCHERING AG)<br>* Ansprüche * | 1,11 | C 07 J 1/00<br>A 61 K 31/565 |
| A | US-A-3 917 829 (W. VOIGT)<br>* Ansprüche * | 1,11 | |
| | | | **RECHERCHIERTE SACHGEBIETE (Int. Cl.5)** |
| | | | C 07 J 1/00 |

Der vorliegende Recherchenbericht wurde für alle Patentansprüche erstellt

| Recherchenort | Abschlußdatum der Recherche | Prüfer |
|---|---|---|
| DEN HAAG | 14-09-1989 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)